**Europäisches Patentamt**

**European Patent Office**

**Office européen des brevets**

(19)

(11) Publication number: **0 191 994**
**B1**

(12) **EUROPEAN PATENT SPECIFICATION**

(45) Date of publication of patent specification: **13.12.89**

(21) Application number: **85309499.3**

(22) Date of filing: **24.12.85**

(51) Int. Cl.⁴: **A 61 L 27/00, A 61 K 37/12, C 08 H 1/06**

(54) Solution for use in viscosurgery and/or as a vitreous substitute.

(30) Priority: **22.01.85 US 693084**

(43) Date of publication of application:
**27.08.86 Bulletin 86/35**

(45) Publication of the grant of the patent:
**13.12.89 Bulletin 89/50**

(84) Designated Contracting States:
**BE CH DE FR GB IT LI NL SE**

(56) References cited:
**EP-A-0 124 659**
**EP-A-0 132 979**
**FR-A-2 335 198**
**FR-A-2 393 581**
**US-A-3 949 073**
**US-A-4 223 984**
**US-A-4 505 855**

(73) Proprietor: **KOKEN CO. LTD.**
**5-18 Shimoochiai 3-chome**
**Shinjuku-ku Tokyo (JP)**

(72) Inventor: **Miyata, Teruo**
**314, 3-6-29 Shimoochiai**
**Shinjuku-ku Tokyo (JP)**
Inventor: **Dunn, Michael W.**
**1073 North Avenue**
**New Rochelle, N.Y. 10804 (US)**

(74) Representative: **Crisp, David Norman et al**
**D. YOUNG & CO. 10 Staple Inn**
**London, WC1V 7RD (GB)**

Courier Press, Leamington Spa, England.

## Description

This invention relates to viscous, transparent, pyrogen-free isotonic solutions for use in viscosurgery and/or as a vitreous substitute.

Solutions of fractionated sodium hyaluronate have been used in ophthalic viscosurgery. The surgical uses of these compositions are described in US Patent 4,141,973 (Balazs); in Pape, L. G., Balazs, E. A. "The Use of Sodium Hyaluronate (Healon) in Human Interior Segment Surgery" Ophalmology 87:699—705 (1980) and in Healon (sodium hyaluronate) A guide to its Uses in Ophthalmic Surgery, D. Miller and R. Stigmann (John Wily & Sons).

Succinylated collagen compositions are known and have been used in contact lenses, US Patent 4,223,984 (Miyata et al). in skin or wound dressings, US Patent 4,294,241 (Miyata), and in thin membranes suitable for ophthalmic drug delivery, US Patent 4,164,559, (Miyata et al). Stabilized collagen gels made from purified tropocollagen have been used as replacements for vitreous in rabbit and monkey eyes. Stenzel, K. H., Dunn, M. W., Rubin A. L. and Miyata, T. "Collagen Gels: Design for a Vitreous Replacement", Science, June 13, 1969 vol. 164 pp. 1282—83. These unmodified collagen gels, however, have proved deficient for they become opaque under pH conditions found within the eye.

In accordance with the present invention there is provided a solution of succinylated atelocollagen suitable for use in ophthalmic viscosurgery or as a vitreous replacement medium comprising atelocollagen succinylated to between 20% to 50% of the total lysine residue and dissolved in a buffered physiologic solution to a concentration of between 0.5% and 5% by weight.

According to one embodiment of the invention, an improved viscoelastic medium is provided to facilitate ophthalmic surgery procedures by preventing tissue damage, inter alia, as a result of its function as a lubricant. The medium also allows more room for a surgical manipulation while insuring post-operative tissue separation which precludes adhesion formation. Ophthalmic surgery performed with the aid of a viscoelastic medium is known in the art as viscosurgery.

According to another embodiment of the invention, the succinylated collagen solution may be used as an improved vitreous replacement suitable for intraocular implantation. The human vitreous humor is a clear gel-like structure located in the posterior part of the eye. In cases of damaged vitreous, replacement of the damaged vitreous with a clear solution of succinylated collagen may aid in restoring sight.

The following non-limiting Examples serve to illustrate the preparation of succinylated collagen for viscosurgery and vitreous replacement in accordance with the invention:—

Example 1 (reference)

Fresh calf skin was split into three layers, epidermal, corium and flesh. One kilogram of the corium layer was minced using a microcutter and then washed with a 5% NaCl solution to remove soluble substances from the corium insoluble collagen. After washing with water, the minced corium collagen was treated with pepsin (pepsin:collagen=0.5:100 in weight) in 20 litres aqueous acidic solution (pH 3) at 20°C for 3 days. During the pepsin treatment the mixture was slowly stirred. The corium insoluble collagen was completely solubilized by the pepsin treatment. Such pepsin treatment of collagen is preferred when preparing a biomaterial because the antigenic portion (telopeptides) of the collagen molecule are eliminated by the pepsin treatment. The resulting collagen (atelocollagen) is superior for medical surgical uses to a collagen untreated with pepsin.

The solubilized pepsin-treated collagen solution was then filtered successively through 1.0, 0.6 and 0.45 μm pore size membranes. The pH of the filtrate was adjusted to 7.5 and the reconstituted collagen fiber (atelocollagen) was collected by centrifuge. The atelocollagen fiber was redissolved in 5 litres of pH 3 aqueous solution and the pH was adjusted to 8 with NaOH solution. The atelocollagen which had dissolved in the acidic medium formed a precipitate when the pH was adjusted to 8 and a milky white atelocollagen suspension was obtained. To this suspension, 40 ml of acetone containing 4 g of succinic anhydride was gradually added under mixing at constant pH 8. To maintain pH 8 during addition of succinic anhydride, 1 N NaOH was gradually added. The reaction mixture was maintained at pH 8 under mixing overnight. During the succinylation reaction milky white atelocollagen precipitate was dissolved to give a clear solution. After the reaction, the pH of the mixture was adjusted to 4.5 to form a precipitate of succinylated atelocollagen. The precipitate was collected by centrifuge and washed with water at pH 4.5, five (5) times to remove any unreacted succinic acid. The washed succinylated atelocollagen was freezed-dried for storage.

To make the final viscous succinylated atelocollagen solution useful for eye surgery, 10 g of freeze-dried succinylated atelocollagen was dissolved in 1 litre of phosphate buffered saline of pH 7.4 which contained 30 m mol $Na_2HPO_4$ and 200 m mol NaCl. The viscosity of the physiological solution was 1.2 Pa.s and it was pyrogen-free.

When the sterile, pyrogen-free succinylated atelocollagen solution thus prepared was implanted into the anterior chamber of a rabbit eye, it showed minimal inflammatory reaction and remained clear, as is required for viscosurgery of the eye. This same viscous solution was used as a vitreous body substitute in a rabbit eye, and remained clear and showed minimal inflammatory reaction, demonstrating its utility as a vitreous substitute.

As will be appreciated by those skilled in the art, the viscosity of a succinylated atelocollagen solution can be controlled by adjusting the concentration of the atelocollagen, i.e., while a 1% solution has a viscosity of 1.2 Pa.s, a 2% solution has a viscosity of approximately 2 Pa.s, and a 3% solution has a viscosity of approximately 10 Pa.s. The concentration of the succinylated atelocollagen in the buffered physiological solution may range from 0.5% to 5% by weight.

Also, instead of sterilization of the succinylated atelocollagen solution with a 0.45 µm millipore filter, ethylene oxide gas (EOG) can be utilized. Freeze-dried succinylated atelocollagen may be conveniently treated with EOG and then dissolved in phosphate buffered saline after complete removal of excess EOG. Such an EOG sterilized succinylated atelocollagen solution is sterile, pyrogen-free, and useful as a viscosurgery material and as a vitreous body substitute.

The degree of succinylation of the E-NH$_2$ group of atelocollagen can be controlled by controlling the amount of succinic anhydride, the pH and the time of reaction. The degree of succinylation of the atelocollagen prepared by the method described above was over 85% and the isoelectric point of the atelocollagen was 4.5. The degree of succinylation can be determined by the TNBS (Trinitrogenen sulfuric acid) method (Kakade, M. L. and Liener, I. E. (1969) Anal. Biochem. 27, 273—280).

Atelocollagen which is succinylated to more than 10% of the total lysine residue (E-NH$_2$) is soluble at physiologic conditions (pH 7.0, under isotonic conditions).

The denaturation temperature (TD) of the succinylated atelocollagen is dependent on the degree of succinylation. Since the TD drops with an increasing degree of succinylation, an upper limit on the desirable degree of succinylation is generally imposed. For example, the TD is 39.5°C at 24% succinylation, 39.2°C at 35% succinylation and 34.0°C at 90% succinylation. A higher TD is of course, preferable for eye use. Therefore, a 20—50% degree of succinylation of the E-NH$_2$ groups is employed in accordance with the invention.

Example 2 (reference)

Preparation of atelocollagen succinylated to varying degrees was accomplished as follows:

To 100 ml of 0.5% atelocollagen solution at pH 3.0 was added 0.1 N NaOH to adjust the pH to 9.0.

To this atelocollagen at pH 9.0, was added 0.1 ml of 1% succinic anhydride in acetone solution, and the pH of the reaction mixture was maintained at pH 9.0 by adding 0.1 N NaOH for 3 hours. The mixture was then dialyzed against water to remove succinic acid. The dialyzed succinylated atelocollagen was freeze-dried, and redissolved in physiological saline, pH 7, at an atelocollagen concentration of 2%. The degree of succinylation of this atelocollagen was 11.3%

Examples 3 and 4

The above procedure was repeated using 0.3 ml and 1 ml of the 1% succinic anhydride acetone solution. The resulting atelocollagen samples had degrees of succinylation of 24% and 35% respectively.

Claims

1. A solution of succinylated atelocollagen suitable for use in ophthalmic viscosurgery or as a vitreous replacement medium comprising atelocollagen succinylated to between 20% to 50% of the total lysine residue and dissolved in a buffered physiologic solution to a concentration of between 0.5% and 5% by weight.

2. A viscoelastic solution which remains transparent when subjected to an intraocular environment, said solution comprising atelocollagen succinylated to between 20% to 50% dissolved in an aqueous buffer physiologic solution to a concentration of between about 0.5% and 5% by weight.

3. A physiologically acceptable solution of the succinylated atelocollagen according to claim 1 in an aqueous medium at a concentration of between 0.5% and 5% by weight.

4. A solution in accordance with claim 3 wherein the concentration of succinylated atelocollagen is such that the viscosity of the solution is suitable for ophthalmic viscosurgery or as a vitreous humor replacement medium.

5. A solution in accordance with either of claims 3 and 4 which is substantially transparent.

Patentansprüche

1. Lösung von succinyliertem Atelokollagen zur Verwendung in der Viskochirurgie oder als Glaskörperersatzmedium enthaltend Atelokollagen, welches bis zu 20 bis 50% der gesamten Lysinreste succinyliert ist und in einer gepufferten physiologischen Lösung in einer Konzentration zwischen 0,5 und 5 Gew.-% gelöst ist.

2. Viskoelastische Lösung, die in intraokularer Umgebung transparent bleibt, enthaltend auf 20 bis 50% succinyliertes Atelokollagen gelöst in einer wäßrigen physiologischen Pufferlösung mit einer Konzentration von zwischen etwa 0,5 und 5 Gew.-%.

3. Physiologisch annehmbare Lösung des succinylierten Atelokollagens nach Anspruch 1 in einem wäßrigen Medium mit einer Konzentration von zwischen 0,5 und 5 Gew.-%.

4. Lösung nach Anspruch 3, worin die Konzentration des succinylierten Atelokollagens derart ist, daß die Viskosität der Lösung für die Augen-Viskochirurgie oder als Glaskörperersatzmedium geeignet ist.

5. Lösung nach einem der Ansprüche 3 und 4, welche im wesentlichen transparent ist.

Revendications

1. Solution d'atélocollagène succinylé appropriée pour une utilisation en viscochirurgie ophtalmique ou comme produit de remplacement de l'humeur vitrée comprenant de l'atélocollagène succinylé à raison de 20 à 50% par rapport au résidu total de lysine et dissous dans une solution physiologique tamponnée à une concentration comprise entre 0,5 et 5% en poids.

2. Solution viscoélastique qui demeure transparente lorsqu'elle est soumise à un environnement intraoculaire, ladite solution comprenant de l'atélocollagène succinylé à raison de 20 à 50% et dissous dans une solution physiologique tamponnée aqueuse à une concentration comprise entre environ 0,5% et 5% en poids.

3. Solution acceptable du point de vue physiologique de l'atélocollagène succinylé selon la revendication 1 dans un milieu aqueux à une concentration comprise entre 0,5% et 5% en poids.

4. Solution selon la revendication 3, dans laquelle la concentration de l'atélocollagène succinylé est telle que la viscosité de la solution est appropriée pour la viscochirurgie ophtalmique ou comme produit de remplacement de l'humeur vitrée.

5. Solution selon l'une des revendications 3 et 4 qui est sensiblement transparente.